(19) Europäisches Patentamt
European Patent Office
Office européen des brevets

(11) Publication number: **0 456 204 A2**

# (12) EUROPEAN PATENT APPLICATION

(21) Application number: **91107466.4**

(51) Int. Cl.5: **A61K 31/38**

(22) Date of filing: **08.05.91**

(30) Priority: **09.05.90 GB 9010332**

(43) Date of publication of application:
**13.11.91 Bulletin 91/46**

(84) Designated Contracting States:
**AT BE CH DE DK ES FR GB GR IT LI LU NL SE**

(71) Applicant: **SANDOZ LTD.**
**Lichtstrasse 35**
**CH-4002 Basel(CH)**
(84) **BE CH DK ES FR GB GR IT LI LU NL SE**

Applicant: **SANDOZ-PATENT-GMBH**
**Humboldtstrasse 3**
**W-7850 Lörrach(DE)**
(84) **DE**

Applicant: **SANDOZ-ERFINDUNGEN**
**Verwaltungsgesellschaft m.b.H.**
**Brunner Strasse 59**
**A-1235 Wien(AT)**
(84) **AT**

(72) Inventor: **Seibel, Markus J.**
**Handschuhsheimer Landstrasse 29**
**W-6900 Heidelberg(DE)**

## (54) Use of benzocycloheptathiophenecarboxylic acid derivatives in the treatment of eosinophile accumulation/activation and connected diseases.

(57) α-[10-Oxy-4H-benzo[4,5]cyclohepta[1,2-b]thiophen-4-ylidene]-carboxylic acids, for example [10-methoxy-4H-benzo[4,5]-cyclophepta[1,2-b]thiophen-4-ylidene]-acetic acid, in particular its (Z)-isomer, their physiologically-hydrolysable and -acceptable esters, as well as pharmaceutically acceptable salts thereof are useful in treating eosinophil accumulation/activation, and are suitable, e.g. for the treatment of obstructive and inflammatory airways desease, for example asthma, as well as other allergic and non-allergic disorders and conditions, for example eosinophil-related disorders of various type.

EP 0 456 204 A2

The present invention relates to a new use, in particular a new pharmaceutical use, for the compound group comprising α-[10-oxy-4H-benzo[4,5]cyclohepta[1,2-b]thiophen-4-ylidene]-carboxylic acids, their physiologically-hydrolysable and -acceptable esters and their pharmaceutically acceptable salts, said compound group being referred to herein collectively as COMPOUNDS OF THE INVENTION.

COMPOUNDS OF THE INVENTION are known and have been described together with processes for their production in European patent publication no. 0 138 765 A (= Application No. 84810475) and equivalents world-wide, including e.g. Australian patent application no. 34145/84 and US patent no. 4 740 518, as well as in UK patent publication no. 2 183 648 A and equivalents world-wide, including e.g. Australian patent application no. 66175/86 and U.S. patent application no. 82875.

As described in said patent references the 4H-benzo[4,5]-cyclohepta[1,2-b]thiophene nucleus of COMPOUNDS OF THE INVENTION may bear substitutents in addition to those specified at the 4- and 10-positions. In particular there may be further substitution in the benzene and/or in the thiophene ring. Thus apart from the substitutents at the 4- and 10-positions, the 4H-benzo[4,5]cyclohepta[1,2-b]-thiophene nucleus may, for example, be substituted, e.g. mono-substituted, in the benzene ring by halogen, e.g. chlorine, or hydroxy, or they may be substituted at the 2-position of the thiophene ring by halogen.

One particular group of COMPOUNDS OF THE INVENTION described and claimed in European patent publication no. 0 138 765 A comprises compounds of formula Ia

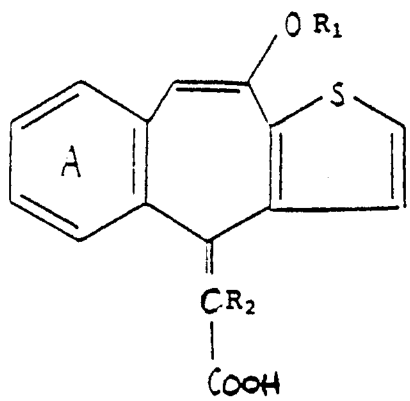

(Ia)

wherein

$R_1$ is hydrogen, $C_{1-4}$alkyl or phenyl-($C_{1-4}$alkyl),

$R_2$ is hydrogen, or $C_{1-4}$alkyl, and

ring A is unsubstituted or halo- or hydroxy-substituted; and their physiologically-hydrolysable and -acceptable esters and their pharmaceutically acceptable salts.

Compounds of the above formula 1a wherein $R_1$ is $C_{1-4}$alkyl, $R_2$ is hydrogen and ring A is unsubstituted or mono-hydroxy or mono-halo (e.g. mono-chloro) substituted, preferably wherein ring A is unsubstituted, and their physiologically-hydrolysable and -acceptable esters and their pharmaceutically acceptable salts, form a preferred sub-group.

A further group of COMPOUNDS OF THE INVENTION described and claimed in UK patent application no. 2 183 648 comprises [2-halo-10-oxy-4H-benzo-[4,5]cyclohepta[1,2-b]thiophen-4-ylidene]acetic acids, their physiologically-hydrolysable and -acceptable esters and their pharmaceutically acceptable salts, for example compounds of formula 1b

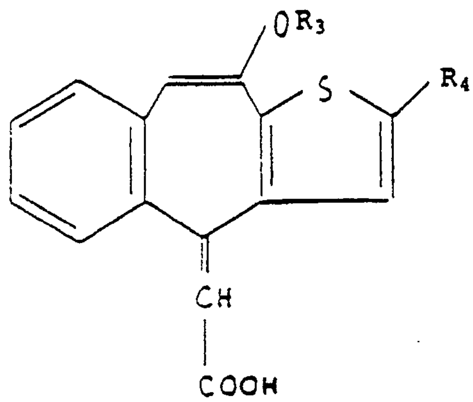

(Ib)

wherein
$R_3$ is hydrogen or $C_{1-4}$alkyl and
$R_4$ is halogen,
and their physiologically-hydrolysable and -acceptable esters and their pharmaceutically acceptable salts.

In accordance with the teachings of said UK patent publication no. 2 183 648 A, compounds of the above formula 1b wherein $R_3$ is $C_{1-4}$alkyl and their physiologically-hydrolysable and -acceptable esters and their pharmaceutically acceptable acid addition salts, form a preferred sub-group.

Compounds of formula 1a and 1b, esters and salts as defined above as well as the above defined sub-groups thereof are also preferred as COMPOUNDS OF THE INVENTION for use in accordance with the present invention, compounds of formula 1a, their esters and salts being especially preferred.

In the compounds of formula 1a and 1b, alkyl groups as $R_1$, $R_2$ and $R_3$ as well as alkyl moieties of phenyl-($C_{1-4}$alkyl) groups as $R_1$ (in formula 1a) may be branched or straight-chain. When $R_1$ or $R_3$ is $C_{1-4}$alkyl, this is preferably methyl. By halogen as $R_4$ (in formula 1b) is meant fluorine, chlorine, bromine or iodine. Preferably $R_4$ is chlorine.

By the term "physiologically-hydrolysable and -acceptable esters", e.g. as applied compounds of formula 1a or 1b, is meant esters in which the carboxylic group is esterified and which are hydrolysable under physiological conditions to yield an alcohol which is itself physiologically acceptable, e.g. non-toxic, at desired dosage levels. Such esters include, e.g. esters with aliphatic alcohols having 1 to 4 carbon atoms.

Pharmaceutically acceptable salts, e.g. of compounds of formula 1a or 1b, include, e.g. alkali metal salts, such as the sodium and potassium salts, as well as alkaline earth metal salts, such as the calcium salts.

It will be appreciated that COMPOUNDS OF THE INVENTION wherein the 10-oxy group is 10-hydroxy, e.g. compounds of formula 1a or 1b wherein $R_1$ or $R_3$ is hydrogen, exist in both keto as well as in enol form, e.g. in the case of compounds of formula 1a and 1b as described in European patent publication no. 0 138 765 A2 and UK patent publication no. 2 183 648 A. It is to be understood that, where tautomeric forms occur, the present invention embraces use of both keto and enol forms, i.e., in so far as COMPOUNDS OF THE INVENTION are defined herein, for convenience, by reference to the enol form only, the invention is not to be understood as being in any way limited by the particular nomenclature or graphic representation employed.

COMPOUNDS OF THE INVENTION, e.g. compounds of formula 1a or 1b, exist in both cis and trans isomeric forms, i.e. as Z and E isomers. The present invention is to be understood as embracing use of both individual cis and trans isomers as well as mixtures thereof. In the present specification and claims cis (Z) and trans (E) isomers are designated in accordance with conventional CIP-nomenclautre [Angew. Chem. 94, 614 81982) and Loc. cit.], as more closely explained, e.g. in the aforementioned European patent publication no. 0 138 765 and UK patent publication no. 2 183 648 A.

In general, for the purposes of the present invention, use of the (Z) isomers of COMPOUNDS OF THE INVENTION is preferred. For use in accordance with the invention, COMPOUNDS OF THE INVENTION are thus preferably in predominantly Z-isomeric form. Most preferably they are in pure or substantially pure Z-isomeric form.

Individual compounds, specifically disclosed in European patent publication no. 0 138 765 A2 and UK patent publication no. 2 183 648 A, suitable for use in accordance with the present invention are:

A) [10-Methoxy-4H-benzo[4,5]cyclohepta[1,2-b]thiophen-4-ylidene]-acetic acid ethyl ester [(Z,E)-isomer

mixture].

B) [7-Chloro-10-methoxy-4H-benzo[4,5]cyclohepta[1,2-b]thiophen-4-ylidene]-acetic acid ethyl ester: B1) (Z,E)-isomer mixture; B2) (Z)-isomer; and B3) (E)-isomer.

C) [6-Hydroxy-10-methoxy-4H-benzo[4,5]cyclohepta[1,2-b]-thiophen-4-ylidene]-acetic acid ethyl ester: C1) (Z,E)-isomer mixture; C2) (Z)-isomer; and C3) (E)-isomer.

D) [10-Methoxy-4H-benzo[4,5]cyclohepta[1,2-b]thiophen-4-ylidene]-acetic acid: D1) (Z,E)-isomer mixture; D2) (Z)-isomer; and D3) (E)-isomer.

E) [10-Methoxy-4H-benzo[4,5]cyclohepta[1,2-b]thiophen-4-ylidene]-acetic acid methyl ester: E1) (Z,E)-isomer mixture; E2) (Z)-isomer; and E3) (E)-isomer.

F) [7-Chloro-10-methoxy-4H-benzo[4,5]cyclohepta[1,2-b]thiophen-4-ylidene]-acetic acid: F1) (Z,E)-isomer mixture; F2) (Z)-isomer; and F3) (E)-isomer.

G) [6-Hydroxy-10-methoxy-4H-benzo[4,5]cyclohepta[1,2-b]-thiophen-4-ylidene]-acetic acid ethyl ester: G1) (Z,E)-isomer mixture; G2) (Z)-isomer; and G3) (E)-isomer.

H) [10-Hydroxy-4H-benzo[4,5]cyclohepta[1,2-b]thiophen-4-ylidene]-acetic acid [(Z,E)-isomer].

J) [2-Chloro-10-methoxy-4H-benzo[4,5]cyclohepta[1,2-b]thiophen-4-ylidene]-acetic acid: J1) (Z,E)-isomer mixture; J2) (Z)-isomer; and J3) (E)-isomer.

K) [2-Chloro-10-methoxy-4H-benzo[4,5]cyclohepta[1,2-b]thiophen-4-ylidene]-acetic acid: K1) (Z)-isomer; and K2) (E)-isomer;

Preferred compounds for use in accordance with the present invention are compounds K1 (herein referred to as COMPOUND K1) and, in particular, D2 (hereinafter referred to as COMPOUND D2). Most preferably COMPOUND K1 and COMPOUND D2 will be used in pure or substantially pure form.

As described in European patent publication no. 0 138 765 A2 and UK patent publication no. 2 183 648, COMPOUNDS OF INVENTION are useful as anti-inflammatory agents for use, for example, in the treatment of arthritis and rheumatic diseases, as well as anti-pyretic and analgesic agents. As further described, e.g. in European patent publication no. 0 319 463 A2 and equivalents world-wide COMPOUNDS OF INVENTION have also been found to be useful in effecting monokine, in particular interleukin-1 release or secretion, inhibition and are consequently indicated for use in the treatment of a variety of further diseases or conditions, including autoimmune diseases such as interstitial lung fibrosis.

In accordance with the present invention it has now surprisingly been found that COMPOUNDS OF INVENTION are also useful in suppressing eosinophil accumulation and/or activation, for example allergen induced eosinophil accumulation/activation in the lung. These properties may, for example, be demonstrated in the following test models:

**EXAMPLE A: SUPPRESSION OF EOSINOPHIL ACCUMULATION - ALLERGEN TREATED ANIMALS**

Guinea-pigs (Dunkin-Hartley, male, 400-600g) are sensitised to allergen (ovalbumin) to give high IgE antibody titres.

Challenge of pre-sensitised animals is effected by administration of nebulized 0.1% ovalbomin solution by inhalation.

Animals are sacrificed with pentobarbital (100mg/kg i.p.) 24 hours after challenge. The trachea is exposed and cannulated and the airway lumen washed by introduction and aspiration of 10ml aliquots (x6) of buffered modified Tyrode solution (composition mM: $NaHC0_3$ 11.9 NaCl 136.9, KCl 2.7, $Na_2HP0_4$ 0.4, glucose 5.6, EDTA 19.8; protein % w/v = gelatin 0.1, BSA 0.5; pH to 7.4 by addition of 2N Na0H). Total fluid recovery generally exceeds 80%.

Cell suspensions are concentrated by low speed centrifugation (200g for 10 mins.) and the resulting cell pellet is re-suspended in 1ml modified Tyrode soln. Total and differential cell counts are made from smears fixed in 100% methanol and stained with Leishman stain. At least 500 cells are counted/smear to differentiate cell types.

Control animals (untreated or receiving placebo) exhibit eosinophil accumulation in the airway lumen ca. 24 hours after challenge. Test substance (i.e. COMPOUND OF THE INVENTION) is administered at dosages of 10.0 - 100mg/kg/day as a solution orally for 6 consecutive days prior to challenge, at 1 hour prior to challenge and seven hours after challenge.

Test animals receiving COMPOUND OF THE INVENTION, e.g. COMPOUND D2 or K1 each in substantially pure form, at the above indicated doses exhibit significant reduction in airway eosinophil accumulation compared with untreated controls. By way of example, COMPOUND D2 in substantially pure form is found to exert significant activity in the subject test model at dosages of 10 and 50mg/kg/day s.c..

**EXAMPLE B: INFLUENCE ON ALLERGEN INDUCED EOSINOPHILIA IN THE GUINEA-PIG FOLLOWING INHALATION**

Male guinea-pigs (Dunkin-Hartley) of ca. 300-400g are sensitized employing 10μg amounts of ovalbumin (3x) and applying the technique described in Sanjar et al., Br. J. Pharmacol., 99, 679-686 (1990).

Immediately before challenge COMPOUND OF THE INVENTION, for example COMPOUND D2 or COMPOUND K1 each in substantially pure form, is administered to sensitized animals by inhalation. Administration is effected via a nose-cone either in dry powder with delivery from a brush feed micronising jet mill powder aerosol generator, as described in Bernstein et al. "Aerosols: Science, Technology and Industrial Application of Airborne Particles", Extended Abstract, eds. B.Y.H. Liu. et. al., Elsevier Science, New York, 1984 or in solution from a nebulizer. Antigen challenge or exposure is then carried out, again in accordance with the methods described by Sanjar et. al. loc cit. at p. 680 column 1, employing 10μg ovalbumin in 0.01% solution. Broncheolar lavage is performed 24 hrs following challenge, as follows:

**1. Airway lavage**

Guinea-pigs are killed by an intraperitoneal injection of pentobarbitone 100mg/kg). The trachea is exposed and cannulated. 10ml of Hank's balanced solution is introduced into the lungs and immediately aspirated by gentle compression of the lung tissue. This procedure is repeated five times. Aspiration fluid is pooled at room temperature in Falcon plastic tubes (60ml). Fluid recovered from the six washes normally exceeds 50ml (85%). The cell suspension is centrifuged (200g for 10 minutes); the supernatant is discarded and the cell pellet resuspended in 1ml of Hank's balanced solution.

**2. Total cell counts**

10μl of the 1ml cell suspension is added to 190μl or 90μl (when cell count is low) of Turk's stain (Merck, Darmstadt, Germany) and total cell numbers are determined in a Neubauer (brightline) hemocytometer. The incidence of cells in the 1ml suspension is calculated by multiplying the total cells enumerated in a square of $1mm^2$ by the original dilution viz.: No. of cells counted x dilution x $10^5$ = no. of cells/ml.

**3. Differential cell counts**

The cell suspension is diluted in Hank's balanced solution to obtain a cell concentration of $10^6$ cells per ml. 50μl of this cell suspension is placed in the cytospin (Cytospin 2, Shandon Co.) tube and overlaid by 200μl of buffer. The cytospin is used at 600rpm for 8 minutes.

Results for groups of animals receiving COMPOUNDS OF THE INVENTION at varying doses are compared with results for control challenged animals receiving no test substance. On administration of COMPOUNDS OF THE INVENTION, for example COMPOUND D2 or COMPOUND K1 each in substantially pure form, clear reduction in total cell, eosinophil and neutrophil counts is observed at dosages of from e.g. 10.0 to 100mg/kg, as compared with control groups.

Utility of COMPOUNDS OF THE INVENTION in accordance with the present invention may also be demonstrated in clinical trials, for example performed as follows:

**1. UTILITY IN OBSTRUCTIVE INFLAMMATORY AIRWAYS DISEASE, E.G. ASTHMA**

1.1 Prophylactic efficacy of COMPOUNDS OF THE INVENTION, for example COMPOUND D2 or COMPOUND K1 each in substantially pure form, may be determined in clinical trials of classic design, e.g. involving subjects exhibiting allergic, atopic bronchoconstrictor response, by administration of test medication or placebo 1 to 2 hours prior to allergen challenge, or by administration of test at regular dosage rates (e.g. 2 or 3x daily) or placebo over a period of 1 to 7 days prior to allergen challenge.

Suitable individual oral dosages, e.g. for COMPOUNDS D2 and K1 in substantially pure form, in such clinical trials comprise, e.g. from ca. 10 to 800mg, e.g. from ca. 50 to 250mg/day, for example at dosages of ca. 25, 50, 200 or 400mg/day orally administered 1x or in divided doses 2 to 3x daily, for example in encapsulated form as hereinafter described in EXAMPLE F.

Both acute (0 to 1 hour) and long-term (up to 12 hour) change in lung function is determined post-medication by measurement of lung function parameters, e.g. as follows:

a) FVC; FEV 0.5; FEV 1.0; FEV 3.0;

b) FEV 0.5/FVC; FEV 1/FVC; FEV 3/FVC;

c) PEF; FEF 25-75%; FEF 75-85%; FEF 25%; FEF 50%; FEF 75%; FEF 0.2-1.2

d) Flow expiratory curve and flow inspiratory curve.

Tolerance of medication and possible occurrence of side effects are also reported.

In addition to monitoring of lung function, blood eosinophil levels are also monitored and the incidence of activated eosinophils and eosinophil secretory proteins determined. In individual studies, subjects are also subjected to lung lavage and the presence of eosinophils and platelets and their respective secretory products determined before and after exposure to allergen.

In clinical trials designed in accordance with the above principles, COMPOUND D2 and COMPOUND K2, each in substantially pure form, provide reduction of eosinophilic response, as well as marked improvement in lung function parameters compared with control subjects receiving placebo only, on administration at dosages indicated. Similar results are obtained with other COMPOUNDS OF THE INVENTION at the same or equivalent dosage-rates.

1.2 Prophylactic efficacy may also be demonstrated in clinical trials involving administration to groups of asthmatic subjects on a regular daily basis over periods from at least 2, preferably 3 months, up to 6 months and more. Initial dosaging for e.g. COMPOUND D2 or COMPOUND K1 in substantially pure form is of the order of from 10 to 800mg, e.g. from ca. 50 to 250mg/day, for example at dosages of ca. 25, 50, 200 or 400mg/day administered, e.g. orally once or in divided dosages 2 to 3x daily with subsequent reduction in appropriate cases to a maintenance dosage of 10 to 400mg/day orally. (Suitable dosage forms are e.g. as hereinafter described in EXAMPLE F). During the course of the trial subjects are monitored continually and all relevant parameters recorded including lung function, alterations in the incidence of activated eosinophils or the release of eosinophil products into the blood or bronchial fluids and, in particular, frequency and severity of asthma exacerbation or attack. Additional bronchodilator therapy is provided on the occasion of any such attack.

Subjects receiving therapy comprising COMPOUND D2 or COMPOUND K2 at dosages indicated above, or other COMPOUNDS OF THE INVENTION at the same or equivalent dosage rates, show marked reduction in frequency of asthma exacerbation or attack, tending, with duration of the trial, to lead to freedom from attack as compared with subjects receiving alternative, non-prophylactic therapy. Where participating subjects are initially dependent on concomitant, bronchodilator, e.g. methylxanthine or $\beta_2$-agonist drug therapy, increasingly reduced need for concomitant therapy is also observed.

**2. UTILITY IN PNEUMOCONIOSIS/BRONCHITIS**

The trial is performed analogously to 1.2 above but with groups of subjects having a history of pneumoconiosis or severe bronchitis, e.g. characterised by events of restricted lung function, for example including difficulty in breathing, wheezing, dyspnea or occasional bronchoconstrictor attack and a record of pneumoconiosis- or bronchitis-related work absenteeism. Suitable subjects include cotton-mill workers exhibiting severe byssionosis. COMPOUND D2 or COMPOUND K1 each in substantially pure form is administered daily at oral dosage rates as set forth under 1.2 above, over periods in excess of 2, preferably in excess of 3, months.

During the course of the trial, subjects are monitored continually and all relevant parameters are recorded, including basal lung function, frequency of attack with impairment of lung function, requirement for other medication and frequency of absenteeism attributable to disease status.

Subjects receiving therapy exhibit overall improvement in basal lung function during the course of the trial accompanied by reduced frequency of attack and absenteeism. In cases where frequent use of alternative medication is recorded at trial entry, reduced need for concomitant medication is also observed as the trial progresses. Similar results are obtainable employing other COMPOUNDS OF THE INVENTION at the same or equivalent dosage rates.

**3. UTILITY IN HYPEREOSINOPHILIA AND EOSINOPHIL-RELATED DISORDERS**

The trial is performed analogously to 1.2 above but with groups of subjects exhibiting elevated eosinophil presence in the blood attributable to eosinophil-related disorder, e.g. accompanying Loffeler's syndrome, eosinophilic pneumonia, eosinophilic fascitis, or attributable to hypereosinophilia.

COMPOUND D2 or COMPOUND K1 in substantially pure form is administered orally at a daily dosage rate of from 10 to 400mg, e.g. from cam. 50 to 250mg/day, for example at dosages of ca. 25, 50 or 200mg, e.g. in dosage form as hereinafter described in EXAMPLE F. The trial is continued for a period of up to 2 to 3 months. Blood samples are collected regularly during the course of the trial and assayed for blood-eosinophil count, incidence of activated eosinophils and levels of eosinophil secretory proteins.

Subjects receiving therapy as described are found to exhibit decreased blood-eosinophil count during the course of treatment accompanied by decreased incidence of activated eosinophils ad eosinophil secretory protein levels. Recorded symptomatology attributable to condition is also found to undergo reversal during treatment. Similar results are obtainable employing other COMPOUNDS OF THE INVENTION at the same or equivalent dosage rates.

In accordance with the foregoing the present invention provides, in a first aspect:

I. A method of treating eosinophil accumulation or activation in a subject in need thereof, which method comprises administering to said subject an effective amount of a COMPOUND OF THE INVENTION.

In the alternative the present invention provides

Ia. A COMPOUND OF THE INVENTION for use in a method as set forth under I above; or

Ib. A pharmaceutical composition comprising a COMPOUND OF THE INVENTION for use in a method as set forth under I above; or

Ic. A COMPOUND OF THE INVENTION for use in the preparation of a pharmaceutical composition for use in a method as set forth under I above.

Having regard to their activity a) in inhibiting airways eosinophil accumulation/activation, and b) in inhibiting acute response in hypersensitive subject following allergen or other challenge, COMPOUNDS OF THE INVENTION are in particular useful in the treatment, especially prophylactic treatment, of obstructive or inflammatory airways disease.

By continued administration, COMPOUNDS OF THE INVENTION may be used to provide advance protection against recurrence of bronchoconstrictor attack consequential to obstructive or inflammatory airways disease, e.g. asthma, or for the control, restriction or reversal of basal status of such disease, e.g. for the control restriction or reversal of basal causes of asthma and asthma attack. The words "treatment" and "treating" as used throughout the present specification and claims are accordingly to be understood as including both symptomatic and, especially prophylactic modes, unless otherwise specified.

In accordance with the foregoing the present invention accordingly also provides:

II. A method for the treatment of obstructive or inflammatory airways disease in a subject in need thereof, which method comprises administering to said subject an effective amount of a COMPOUND OF THE INVENTION; for example:

A method for the prophylactic treatment of obstructive or inflammatory airways disease (e.g. for advance protective treatment against acute airways obstruction, for example bronchospasm, e.g. as occurring in the symptomatology of diseases, disorders or conditions hereinafter set forth) in a subject in need thereof, which method comprises administering to said subject a prophylactically effective amount of a COMPOUND OF THE INVENTION.

In the alternative the present invention provides:

IIa. A COMPOUND OF THE INVENTION for use in the treatment of obstructive or inflammatory airways disease, e.g. for use in a method as set forth under II above; or

IIb. A pharmaceutical composition comprising a COMPOUND OF THE INVENTION for use in a method as set forth under II above; or

IIc. A COMPOUND OF THE INVENTION for use in the preparation of a pharmaceutical composition for use in a method as set forth under II above.

The method of the present invention as defined under II above is, in particular, applicable to the treatment of asthma of whatever type or genesis. It is applicable to both intrinsic and, especially, extrinsic asthma. It is especially applicable to the treatment of allergic asthma, whether atopic, (i.e. IgE mediated) or non-atopic, as well as, e.g. bronchitic asthma, exercise induced asthma, occupational asthma, asthma induced following bacterial infection and other non-allergic asthmas. The method of the invention is, in particular, suitable for the treatment of asthma concomitant to or associated with other inflammatory autoimmune diseases, for example severe arthritis. Treatment of asthma is also to be understood as embracing treatment of subjects, e.g. of less than 4 or 5 years of age, exhibiting wheezing symptoms, in particular at night, and diagnosed or diagnosable as "wheezy infants", an established patient category of major medical concern and now more correctly identified as incipient or early-phase asthmatics. (For convenience of definition, this particular asthmatic condition is referred to as "wheezy-infant syndrome".)

The method of the present invention as defined under II above is also applicable to the treatment of pneumoconiosis (an inflammatory, commonly occupational, disease of the lungs, frequently accompanied by airways obstruction, whether chronic or acute, and occasioned by repeated inhalation of dusts) of whatever type or genesis, including, for example, aluminosis, anthracosis, asbestosis, chalicosis, ptilosis, siderosis, silicosis, tabacosis and in particular, byssinosis.

The method of the present invention as defined under II above, is also applicable to the treatment of bronchitis or, more especially, the treatment of chronic or acute airways obstruction, for example, dyspnea, associated therewith. In this respect the present invention is applicable to the treatment of bronchitis of whatever type or genesis, including, for example, acute bronchitis, arachidic bronchitis, catarrhal bronchitis, chronic bronchitis, croupous bronchitis, phthioid bronchitis and so forth.

Having regard to activity of COMPOUNDS OF THE INVENTION in suppressing eosinophil accumulation/activation, the present invention also provides:

III. A method for the treatment of disease characterised by or having an aetiology comprising morbid eosinophil accumulation and/or activation, in a subject in need thereof which method comprises administering to said subject an effective amount of COMPOUND OF THE INVENTION;

or, in the alternative:

IIIa. A COMPOUND OF THE INVENTION for use in a method as set forth under III above; or

IIIb. A pharmaceutical composition comprising a COMPOUND OF THE INVENTION for use in a method as set forth under III above; or

IIIc. A COMPOUND OF THE INVENTION for use in the preparation of a pharmaceutical composition for use in a method as set forth under III above.

Diseases as defined under III above include, in particular, hypereosinophilia and the eosinophil-related disorders.

Hypereosinophilia is a distinct condition or status of varied aetiology characterised by chronic, morbid eosinophil presence in the body tissues generally. The eosinophil-related disorders comprise a distinct and extensively documented indication group, commonly occurring concomitant to another, primary disease or condition. [For more detailed discussion see, e.g. Schatz et al, Medical Clinics of North America 65(5) 1055-1071 (1981) and Ottesen et al "Allergy, Principles and Practice", Eds. E. Middleton, C. Reed and S. Ellis, 584-632 (1987).] The group includes eosinophil related disorders of the airways (involving morbid eosinophilic infiltration of pulmonary tissues) as well as of other organs and tissues including, for example, the skin, eye, nasal passages and the urinary tracts.

Eosinophil-related disorders to which the present invention is applicable include those concomitant to atopy or atopic reactions in general (e.g. atopic conditions such as rhinitis, conjunctivitis etc. as set forth below) as well as non-atopic eosinophil related disorders.

Disorders of the airways to which the present invention is applicable include hypereosinophilia as well as, for example, eosinophil-related disorders of the airways consequential or concomitant to Löffler's syndrome, eosinophilic pneumonia, parasitic (in particular metazoan) infestation (including tropical eosinophilia), bronchopulmonary aspergillosis, polyarteritis nodosa (including Churg-Strauss syndrome) as well as eosinophil-related disorders affecting the airways occasioned by drug reaction.

Other eosinophil related disorders to which the present invention is applicable include eosinophilia consequential or concomitant to eosinophilic gastroenteritis, Heiner's syndrome, atopic dermatitis, urticaria or angioderma (allergic, recurrent or prolonged), ichthyosis, exfoliative dermatitis or pityriasis rubra, urticaria pigmentosa or mastocytoma, toxic epidermal necrolysis (drug related), dermatitis herpetiformis, allergic rhinitis, hyperplastic sinusitis, interstitial nephritis (drug related), interstitial cystitis, choleostatic hepatotoxicity (drug related), allergic conjunctivitis, vernal conjunctivitis, eosinophilic fascitis, hypersensitivity angiitis, serous myocarditis or endomyocardial fibrosis, Wiscott-Aldrich syndrome, selective IgA deficiency with atopy, eosinophilic leukaemia and eosinophilic granuloma.

As will be appreciated the present invention is directed primarily to the treatment of hypereosinophilia or eosinophil related disorders as such. Where, however, eosinophil related disorders are concomitant to atopy, for example to any of the atopic diseases or conditions specifically recited above including atopic or allergic forms of dermatitis, urticaria, angioderma, rhinitis, conjunctivitis and gastrointestinal allergies, the present invention may equally be applicable to the treatment of eosinophil related disorder as and integral or basal component thereof. The present invention thus also provides means for the treatment (e.g. symptomatic or prophylactic treatment) of atopy, including each of the said recited atopic diseases or conditions, as such. In treating eosinophil related disorders concomitant to non-atopic diseases or conditions on the other hand, the COMPOUNDS OF THE INVENTION will more commonly be administered together with other medication for treatment of the disease or condition with which eosinophilia is associated. Thus in the treatment of eosinophilia consequential to parasitic infection, use will generally be in conjunction with other anti-parasitic drug therapy.

Dosages employed in practicing the present invention will of course vary depending, e.g. on the particular condition to be treated, the particular COMPOUND OF THE INVENTION employed, the mode of administration and the therapy desired. In general however, for the above mentioned uses, in particular for the symptomatic and/or prophylactic treatment of obstructive or inflammatory airways disease, for example asthma, satisfactory results are achieved at oral dosages of from about 10mg to about 800mg per day, preferably from about 50 to about 200 or 400mg per day, conveniently administered once or in divided doses 2 to 4x/day or in sustained release form. Unit dosage forms for oral administration thus suitably comprise from about 25mg to about 400 or 800mg (e.g. 25, 50, 100 or 200mg) of COMPOUND OF THE INVENTION, e.g. COMPOUND D2 or COMPOUND K1 together with a pharmaceutically acceptable diluent or carrier therefor.

COMPOUNDS OF THE INVENTION may be administered in similar manner to known standards, e.g. theophylline, for use in the recited indications, e.g. orally in unit dosage form, e.g. in the form of tablets or capsules. They may also be administered by inhalation, i.e. by the pulmonary route, e.g. by delivery in dry powder or in solution from an appropriate dispenser device, e.g. atomizer, dry-powder inhalation device or the like, as known in the art.

The following is illustrative of the preparation of solid compositions in accordance with the invention.

**EXAMPLE C: Production of solid compositions for oral application**

Tablets or capsules may contain the active agent in admixture with conventional pharmaceutically acceptable excipients, e.g. inert diluents such as calcium carbonate, sodium carbonate, lactose and talc, granulating and disintegrating agents, e.g. starch and alginic acid, flavouring, colouring and sweetening agents, binding agents, e.g. starch, gelatin and acacia, and lubricating agents, e.g. magnesium stearate, stearic acid and talc.

The following example is illustrative of the preparation of capsule forms useful in the method of the present invention on administration from 1 to 4x daily:

COMPOSITION 1

| INGREDIENTS | | WT./DOSE |
|---|---|---|
| Active ingredient, e.g. COMPOUND D2* or K1* above in substantially pure form | | 200.00 mg |
| Lactose (200 mesh) | | 109.75 mg |
| Corn starch | | 35.00 mg |
| Silicon dioxide (Aerosil 200) | | 1.75 mg |
| Magnesium stearate | | 3.50 mg |
| | TOTAL | 350.00 mg |

The active ingredients are SIEVED, intimately admixed employing conventional galenic procedures, granulated, dried and filled into hard gelatin capsules and the capsules sealed. Capsule weight = 97.0 mg: total weight for filled capsule = 447.0 mg.

| INGREDIENTS | | WT./DOSE (mg) | |
|---|---|---|---|
| | | COMPOSITION 1 | 2 |
| Active ingredient, e.g. COMPOUND D2* or K1* | | 50.00 | 100.00 |
| Lactose (200 mesh) | | 259.75 | 209.75 |
| Corn Starch | | 35.00 | 35.00 |
| Silicon dioxide (Aerosil 200) | | 1.75 | 1.75 |
| Magnesium stearate | | 3.50 | 3.50 |
| | TOTAL | 350.00 | 350.00 |

(*Both compounds are preferably employed in free acid form)

In accordance with the foregoing the present invention also provides

IV. A method for the preparation of a pharmaceutical composition as defined under any one of Ib, IIb and IIIb above, which method comprises intimately admixing a COMPOUND of the invention together with one or more pharmaceutically acceptable solvents or carriers therefore and, when desired compounding said composition in unit dosage form e.g. as a capsule, tablet, dragée or the like.

COMPOUNDS OF THE INVENTION are well tolerated at dosages required for use in accordance with the present invention.

Thus established $LD_{50}$ values for the COMPOUND D2 in substantially pure form in mice and rats after 14 days p.o. and 7 days i.v. are: in mice, p.o 1623 mg/mg, i.v. 163 mg/kg; in rats, p.o. 1721 mg/kg, i.v. 50 mg/kg.

In beagle dogs the same compound is found to be generally well tolerated when administered at high dose levels of up to 200 mg/kg/day P.O for 26 weeks.

In clinical trials COMPOUND D2 is found to be well tolerated with no significant side effect occurrance at oral dosage rates of 50, 200 and 400mg/day.

For COMPOUND K1 in substantially pure form, in pilot toxicological studies in the beagle dog, no pathological effects are observed on administration of doses of 150 and 200 mg/kg p.o. in gelatin capsules over 5 weeks and in olive oil from week 6 to week 8 on.

Pharmaceutically acceptable acid addition salt forms exhibit the same or similar levels of tolerability/activity as free acids.

## Claims

1. A method of treating eosinophil accumulation or activation, which method comprises administering an α-[10-oxy-4H-benzo[4,5]cyclohepta[1,2-b]thiophen-4-yliden]-carboxylic acid or a physiologically-hydrolysable and -acceptable ester or a pharmaceutically acceptable salt thereof.

2. A method of treating obstructive or inflammatory airways disease which method comprises administering an α-[10-oxy-4H-benzo[4,5]cyclohepta[1,2-b]thiophen-4-yliden]-carboxylic acid or a physiologically-hydrolysable and -acceptable ester or a pharmaceutically acceptable salt thereof.

3. A method according to claim 2 for the treatment of asthma, pneumoconiosis or bronchitis.

4. A method of treating hypereosinophilia or an eosinophil-related disorder which method comprises administering an α-[10-oxy-4H-benzo[4,5]cyclohepta-[1,2-b]thiophen-4-yliden]-carboxylic acid or a physiologically-hydrolysable and -acceptable ester or a pharmaceutically acceptable salt thereof.

5. A method according to claim 4 for the treatment of an eosinophil-related disorder concomitant to atopy or for the treatment of atopic reaction.

6. A method of treating eosinophil-related disorder of the airways, rhinitis or conjunctivitis which method comprises administering an α-[10-oxy-4H-benzo[4,5]-cyclohepta[1,2-b]thiophen-4-yliden]-carboxylic acid or a physiologically-hydrolysable and -acceptable ester or a pharmaceutically acceptable salt thereof.

7. A method according to any one of claims 1 to 6 comprising administration of the (Z)-isomer of α-[10-methoxy-4H-benzo[4,5]cyclohepta[1,2-b]-thiophen-4-yliden]-acetic acid or a pharmaceutically acceptable acid addition salt thereof; or of the (Z)-isomer of α-[2-chloro-10-methoxy-4H-benzo[4,5]-cyclohepta-[1,2-b]thiophen-4-ylidene]- acetic acid or a pharmaceutically acceptable salt thereof.

8. Use of an α-[10-oxy-4H-benzo[4,5]cyclohepta[1,2-b]-thiophen-4-yliden]-acetic acid or a physiologically-hydrolysable and -acceptable ester or pharmaceutically acceptable salt thereof in a method of treatment as defined in any one of claims 1 to 7.

9. A pharmaceutical composition comprising an α-[10-oxy-4H-benzo[4,5]cyclohepta[1,2-b]thiophen-4-yliden]-acetic acid or a physiologically-hydrolysable and -acceptable ester or pharmaceutically acceptable salt thereof for use in a method of treatment as defined in any one of claims 1 to 7.

10. Use of an α-[10-oxy-4H-benzo[4,5]cyclohepta[1,2-b]-thiophen-4-yliden]-acetic acid or a physiologically-hydrolysable and -acceptable ester or pharmaceutically acceptable salt thereof for use in the preparation of a pharmaceutical composition for use in a method of treatment as defined in any one of claims 1 to 7.